# EUROPEAN PATENT APPLICATION

(11) **EP 2 251 688 A1**
(43) Date of publication of application: **17.11.2010**
(21) Application number: 10008588.5
(22) Date of filing: 11.10.2006
(51) Int. Cl.: G01N 33/50, G01N 33/574

(54) **EGFR dependent modulation of chemokine expression and influence on therapy and diagnosis of tumors and side effects thereof**

(30) Priority: 11.10.2005 EP 05022127
(62) Divisional of application: 06806198.5
(71) Applicant: Merck Patent GmbH, 64293 Darmstadt (DE)
(72) Inventor: Sutter, Arne, 64367 Muehltal (DE); Behrens, Joyce, 81667 München (DE)

(57) **Abstract**

The invention relates to diagnosis and therapy of tumors utilizinging the epidermal growth factor (EGFR) by means of chemical inhibitors or monoclonal antibodies. The invention relates also to skin irritations, preferably skin rash, in conjunction and associated with the treatment of tumor cells that utilize EGF receptor with anti-cancer agents. The invention is also directed to methods of predicting the efficiency of a tumor therapy / tumor response in a patient based on the treatment with EGFR inhibitors, especially anti-EGFR antibodies. The invention further relates to a method of determining the optimum dose of an anti-cancer agent in EGFR related tumor therapy.

## Description

This application is a divisional application of EP 06 806 198.5 filed on 11.10.2006.

The invention relates to diagnosis and therapy of tumors utilizing the epidermal growth factor (EGFR) by means of chemical inhibitors or monoclonal antibodies. The invention relates also to skin irritations, preferably skin rash, in conjunction and associated with the treatment of tumors that utilize EGF receptor with anti-cancer agents. The invention is also directed to methods of predicting the efficiency of a tumor therapy / tumor response in a patient based on the treatment with EGFR inhibitors, especially anti-EGFR antibodies. The invention further relates to a method of determining the optimum dose of an anti-cancer agent in EGFR related tumor therapy. The invention further relates to methods of early stage monitoring of the efficiency of EGFR related cancer therapy by means of EGFR inhibitors, and of the likelihood of occurrence of skin rash as side effect disease in conjunction with said therapy. Finally the invention is directed to the use of chemokines, which are up- or down-regulated during cancer treatment by means of an anti-cancer agent, as a diagnostic marker or as leads for the identification of noval targets for tumor therapeutics..

EGFR, encoded by the erbB1 gene, has been causally implicated in human malignancy. In particular,increased expression of EGFR has been observed in breast, bladder, lung, head, neck and stomach cancer as well as glioblastomas. Increased EGFR receptor expression is often associated with increased production of the EGFR ligand, transforming growth factor alpha (TGF-a), by the same tumor cells resulting in receptor activation by an autocrine stimulatory pathway (Baselga and Mendelsohn, *Pharmac. Ther.* 64:127 (1994)).

The EGF receptor is a transmembrane glycoprotein which has a molecular weight of 170.000, and is found on many epithelial cell types. At present seven EGFR ligands are known which upon receptor binding protect tumor cells from apoptosis stimulate cell proliferation and tumor cell invasiveness. These growth factors do not bind to HER2, HER3 and HER4 the other three members of the EGFR family which can engage with the EGFR in forming heterodimers (Riese and Stern,Bioassays 20: 41-48 (1998); Kochupurakkal J Biol. Chem. 280:8503-8512 (2005))

The HER receptor network might integrate not only its own inputs but also heterologous signals, including hormones, lymphokines, neurotransmitters and stress inducers.

A number of murine and rat monoclonal antibodies against EGF receptor have been developed and tested for their ability inhibit thegrowth of tumor cells in vitro and in vivo (Modjtahedi and Dean, 1994, J. Oncology 4, 277).

Humanized monoclonal antibody 425 (hMAb 425, US 5,558,864; EP 0531 472) and chimeric monoclonal antibody 225 (cMAb 225), both directed to the EGF receptor, have shown their efficacy in clinical trials. The C225 antibody (Cetuximab) was demonstrated to inhibit EGF-mediated tumor cell growth in vitro and to inhibit human tumor formation in vivo in nude mice. The antibody as well as in general all anti-EGFR antibodies act mostly in synergy with certain chemotherapeutic agents (i.e., doxorubicin, adriamycin, taxol, and cisplatin) to eradicate human tumors in vivo in xenograft mouse models (see, for example, EP 0667165). Ye et al. (1999, Oncogene 18, 731) have reported that human ovarian cancer cells can be treated successfully with a combination of both chimeric MAb 225 and humanized MAb 4D5 which is directed to the HER2 receptor.

Besides anti-ErbB antibodies there are numerous small chemical molecules which are known to be potent inhibitors of ErbB receptor molecules mostly blocking ATP binding sites of the receptor. The term *"tyrosine kinase antagonist*/*inhibitor"* refers according to this invention to natural or synthetic agents that are enabled to inhibit or block tyrosine kinases, receptor tyrosine kinases included. Thus, the term includes per se ErbB receptor antagonists / inhibitors as defined above. With exception of the anti-ErbB receptor antibodies mentioned above and below, more preferable tyrosine kinase antagonist agents under this definition are chemical compounds which have shown efficacy in mono- drug therapy for eg breast and prostate cancer. Suitable indolocarbazole-type tyrosine kinase inhibitors can be obtained using information found in documents such as US patents 5,516,771; 5,654,427; 5,461,146; 5,650,407. US patents 5,475,110; 5,591,855; 5,594,009 and WO 96/11933 disclose pyrrolocarbazole-type tyrosine kinase inhibitors and prostate cancer. One of the earliest anti-cancer agents in this context is gefitinib (IRESSA^{®}, Astra Zeneca), which is reported to possess therapeutic efficacy in patients with non-small cell lung cancer (NSCLC) as well as advanced head and neck cancer.

The term "utilization" or "utilize" in context with the EGF receptor has two connotations:
(i) it reflects the fact that the receptor is engaged in signaling. EGFR expression is a necessary but not sufficient precondition for signaling to occurr. Availability and quality of available ligands is as important. Accordingly the degree of receptor expression often does not directly correlate with receptor utilization.
(ii) reflects the fact that the tumor critically depends on the utilization of EGFR.

A number of agents that target this receptor are in use or in development, including monoclonal antibodies (such as cetuximab) and tyrosine kinase inhibitors (such as erlotinib and gefitinib). The most common adverse effect common to EGFR inhibitors is an acne-form rash, usually on the face and upper torso. Skin rash occurs in 45-100% of patients, with a rapid onset in the majority of patients, detectable after approximately 7-10 days of treatment and reaching a maximum after 2-3 weeks (Robert et al., Lancet Oncol. 491, 2005). A positive association of the intensity of rash and treatment response and/or survival has been shown for some agents (including cetuximab and erlotinib), making rash a potential surrogate marker of anti-tumoral activity (Perez-Soler and Saltz, J. Clin. Oncol., 23:5235, 2005).

The mechanisms underlying skin rash remain obscure. In adults, EGFR is primarily expressed in proliferating, undifferentiated keratinocytes of the basal layer of the epidermis and the outer root sheet of the hair follicle (Nanney et al., J. Invest. Dermatol. 83:385, 1984). Alterations in EGFR expression and activity have been linked to abnormal epidermal growth and differentiation (Murillas R et al., EMBO J 1995; Sibilia M et al.,Cell 2000; King LE et al., J Invest Dermatol. 1990).

Keratinocytes are stratified, squamous, epithelial cells which comprise skin and mucosa, including oral, esophageal, corneal, conjunctival, and genital epithelia. Keratinocytes provide a barrier between the host and the environment. They prevent the entry of toxic substances from the environment and the loss of important constituents from the host. Keratinocytes differentiate as they progress from the basal layer to the skin surface. The normal turnover time for keratinocytes is around 30 days but epidermal turnover may be accelerated in some skin diseases such as psoriasis.

Pathological analyses of skin biopsies of patients revealed that EGFR blockade leads to thinning of the stratum corneum and promotes infiltration of inflammatory cells (including neutrophils and T-lymphocytes) into the dermal tissue, particularly the hair follicle (Robert et al., Lancet Oncol. 6:491, 2005; Van Doorn et al., Br. J. Dermatol. 147:598, 2002). Furthermore, signal transduction pathways associated with EGFR were inhibited in the skin suggesting that anti-EGFR therapy has direct effects on epidermal physiology. For example, gefitinib, a small chemical compound (Iressa® ) causes up-regulation of growth arrest- and maturation-marker in the basal layer of the epidermis. Therefore, it may be possible that cell cycle arrest and maturation of keratinocyte cause skin rash since altered differentiation of keratinocytes may lead to follicular occlusions as observed in patients (Albanell et al., J. Clin. Oncol. 20:110, 2002). Alternatively, it is suggested that the development of skin rash may be a direct consequence of altered chemokine expression patterns in the skin analogue to the suggestion made that EGFR functions as negative feed back regulator to prevent excessive inflammation in chronically inflamed skin in vivo (Mascia F et al., Am J Pathol. 2003).

Chemokines are a family of structurally related glycoproteins with potent leukocyte activation and/or chemotactic activity. They are 70 to 90 amino acids in length and approximately 8 to 10 kDa in molecular weight. Most of them fit into two subfamilies with four cysteine residues. These subfamilies are base on whether the two amino terminal cysteine residues are immediately adjacent or separated by one amino acid. The chemokines, also known as CXC chemokines, contain a single amino acid between the first and second cysteine residues; β, or CC, chemokines have adjacent cysteine residues. Most CXC chemokines are chemoattractants for neutrophils whereas CC chemokines generally attract monocytes, lymphocytes, basophils, and eosinophils. There are also 2 other small sub-groups. The C group has one member (lymphotactin). It lacks one of the cysteines in the four-cysteine motif, but shares homology at its carboxyl terminus with the C-C chemokines. The C chemokine seems to be lymphocyte specific. The fourth subgroup is the C-X3-C subgroup. The C-X₃-C chemokine (fractalkine/neurotactin) has three amino acid residues between the first two cysteine. It is tethered directly to the cell membrane via a long mucin stalk and induces both adhesion and migration of leukocytes.

The invention is based on the principal finding that the treatment of EGFR related tumors by means of anti-cancer agent, preferably EGFR inhibitors, causes specific modulations of the chemokine pattern in the skin tissue as well as in the respective tumor tissue or in serum of a patient. The chemokines in said tissue or serum may be down- or up-regulated dependent on the nature and quantity of the anti-cancer agent used in the therapy.

To date there are no clear recommendations for effective rash management during EGFR related tumor therapy, although optimal management will be important especially when EGFR inhibitors are to be used earlier in disease, at higher doses, and/or for longer periods. Based on the present results it is suggested firstly, that modulated chemokine expression patterns in the skin represent useful markers to predict skin rash in patients at early time points during the anti-cancer treatment, enabling clinicians to counteract rash before it can be seen.

Secondly, it is suggested that modulated chemokine expression patterns in the skin are more reliable surrogate markers of effective target inhibition (and thereby possibly also clinical outcome) than skin rash, as in addition to chemokine modulation, skin rash depends on the patient's individual immune system. Thus, patients can be analyzed within the 1^{st} week of treatment to pinpoint patients unlikely to benefit from anti-EGFR therapy and thereby enabling clinicians to change to alternative therapies.

Furthermore, it is suggested that specific agents, such as chemokine receptor blocking agents that interfere with chemokine mediated chemoattraction induced by EGFR blockade may represent novel therapeutics to manage skin disease side effects of EGFR related tumor therapy. These agents should preferentially used topical as their effects on the skin may be mirrored in the tumor.

In summary the invention relates to the following issues:
(1) A method of predicting outbreak and intensity of a skin irritation associated or correlated with cancer therapy in a patient, the method comprising:
   (i) determining in a first skin tissue probe the expression pattern of chemokines with standard methods, wherein the probe is taken from a patient before starting treatment with an anti-cancer agent, which is directed against tumor cells that utilizes epidermal growth factor receptor (EGFR),
   (ii) determining in a second skin probe derived from said patient the expression pattern of chemokines, wherein the probe is taken at a time after having started the treatment with said anti-cancer agent,
   (iii) optionally determining in a third and further skin probe the chemokine expression pattern, wherein the probe is taken from the patient at a later time than the respective precursor probe of step (ii),
   (iv) comparing the respective chemokine expression patterns of the skin probes of step (ii) and optionally (iii) with the expression pattern of the skin probe of step (i), and determining thereof which chemokines have been changed in quality and/ or quantity in probe (ii) and (iii) relative to the cemokine pattern of the reference probe of (i) or the respective precursor probe;
   (v) predicting from the changes in the chemokine pattern the intensity and outbreak at a later time of the skin disease triggered by the treatment with said anti-cancer agent.
(2) A method of (1), wherein said skin irritation is skin rash.
(3) A method of predicting the tumoral response of a patient suffering from cancer to the treatment with an anti-cancer agent, the method comprising:
   (i) determining in a first tissue probe the expression pattern of chemokines with standard methods, wherein the probe is taken from a patient before starting treatment with an anti-cancer agent, which is directed against tumor cells that utilizes epidermal growth factor receptor (EGFR),
   (ii) determining in a second tissue probe derived from said patient the expression pattern of chemokines, wherein the probe is taken at a time after having started the treatment with said anti-cancer agent,
   (iii) optionally determining in a third and further tissue probe the chemokine expression pattern, wherein the probe is taken from the patient at a later time than the respective precursor probe of step (ii),
   (iv) comparing the respective chemokine expression patterns of the tissue probes of step (ii) and optionally (iii) with the expression pattern of the tissue probe of step (i), and determining thereof which chemokines have been changed in quality and/ or quantity in probe (ii) and (iii) relative to the cemokine pattern of the reference probe of (i) or the respective precursor probe;
   (v) predicting from the changes in the chemokine pattern of said tissue probes the likelihood and intensity of the tumoral response of the patient to the treatment with said anti-cancer agent.
(4) A method of determining the optimum dose of an anti-cancer agent for the treatment of cancer in a patient, the method comprising:
   (i) determining in a first tissue probe the expression pattern of chemokines with standard methods, wherein the probe is taken from a patient before starting treatment with an anti-cancer agent, which is directed against tumor cells that utilizes epidermal growth factor receptor (EGFR),
   (ii) determining in a second tissue probe derived from said patient the expression pattern of chemokines, wherein the probe is taken at a time after having started the treatment with said anti-cancer agent,
   (iii) optionally determining in a third and further tissue probe the chemokine expression pattern, wherein the probe is taken from the patient at a later time than the respective precursor probe of step (ii),
   (iv) comparing the respective chemokine expression patterns of the tissue probes of step (ii) and optionally (iii) with the expression pattern of the tissue probe of step (i), and determining thereof which chemokines have been changed in quality and/ or quantity in probe (ii) and (iii) relative to the cemokine pattern of the reference probe of (i) or the respective precursor probe;
   (v) determining the dosis of the anti-cancer agent to be administered to the patient according to the changes in the chemokine pattern of said tissue probes, and optionally
   (vi) repeating steps (i) - (v) in order to optimize the dosis of the anti-cancer agent to be administered to the patient.
(5) A method of (3) or (4), wherein said probe is derived from tumor tissue.
(6) A method of (3) or (4), wherein said probe is derived from skin tissue.
(7) A method of (1 - 6), wherein the probe of step (ii) is taken within 1 - 10 days after onset of the treatment with said anti-cancer agent.
(8) A method of (7), wherein the probe of step (ii) is taken within 5 - 7 days after onset of the treatment with said anti-cancer agent.
(9) A method of (1 - 8), wherein the anti-cancer agent is an EGFR inhibitor.
(10) A method of (9), wherein the EGFR inhibitor is an anti-EGFR antibody
(11) A method of (10), wherein the anti-EGFR antibody is Mab c225 (cetuximab) or Mab h425 (EMD72000, matuzumab).
(12) A method of (1 - 11), wherein the treatment with the anti-cancer agent causes an increased expression of chemokines compared to the non-treated patient.
(13) A method of (1 - 11), wherein the treatment with the anti-cancer agent causes a reduced expression of chemokines compared to the non-treated patient.
(14) A method of (1 - 13), wherein at least one of the following chemokines are involved: IL-8, MCP-1, RANTES and IP-10.
(15) An in-vitro method of early-stage monitoring of the efficiency of the therapy of cancer that utilizes EGFR in a patient by determining the chemokine pattern in probes of skin tissue and / or tumor tissue and / or serum of the tumor patient before starting and during the first 1 - 10 days of treatment with an anti-cancer agent.
(16) An in-vitro method of early-stage monitoring of the occurrence of skin irritation in conjunction with the therapy of cancer that utilizes EGFR in a patient by determining the chemokine pattern in probes of skin tissue of the tumor patient before starting and during the first 1 - 7 days of treatment with an anti-cancer agent.
(17) A method of (15 or 16), wherein the anti-cancer agent is an EGFR inhibitor.
(18) A method of (17), wherein the EGFR inhibitor is an anti-EGFR antibody
(19) A method of (18), wherein the anti-EGFR antibody is Mab c225 (cetuximab) or Mab h425 (EMD72000, matuzumab).
(20) A method of (15 - 19), wherein the treatment with the anti-cancer agent causes an increased expression of chemokines compared to the non-treated patient.
(21) A method of (15 - 19), wherein the treatment with the anti-cancer agent causes a reduced expression of chemokines compared to the non-treated patient.
(22) A method of (15 - 21), wherein at least one of the following chemokines are involved: IL-8, MCP-1, RANTES and IP-10.
(23) Use of chemokines, which are up- or down-regulated in-vivo during cancer treatment with an anti-cancer agent, as a diagnostic marker in-vitro for determining the efficiency of said treatment, and / or the likelihood of the occurrence of skin irritations accompanied by said treatment, wherein said cancer utilizes EGFR and said anti-cancer agent is an EGFR inhibitor.
(24) Use of chemokines, which are up- or down-regulated in-vivo during cancer treatment with an anti-cancer agent, for identification of a target upstream of said chemokine expression, suitable for the development and manufacture of a medicament targeting said target for the treatment of cancer that utilizes EGFR in the patient solely or in combination with said anti-cancer agent, wherein said anti-cancer agent is an EGFR inhibitor.
(25) Use of (23) or (24), wherein said anti-cancer agent is Mab c225 (cetuximab) or Mab h425 (EMD72000, matuzumab).

The current invention has shown by experimental work that blocking EGF receptor, for example, with monoclonal antibodies, such as cetuximab (mAb c225) or mAb h425 (matuzumab) or tyrosine kinase inhibitors (gefitinib, Iressa®), interferes with EGFR-dependent signaling cascades in primary keratinocytes. In these experiments keratinocytes were treated with different concentrations of anti-EGFR inhibitors followed by treatment with or without TGF alpha or TNF alpha for, approximately, 24 h. Effects of anti-EGFR inhibitors were evaluated using Western blotting.

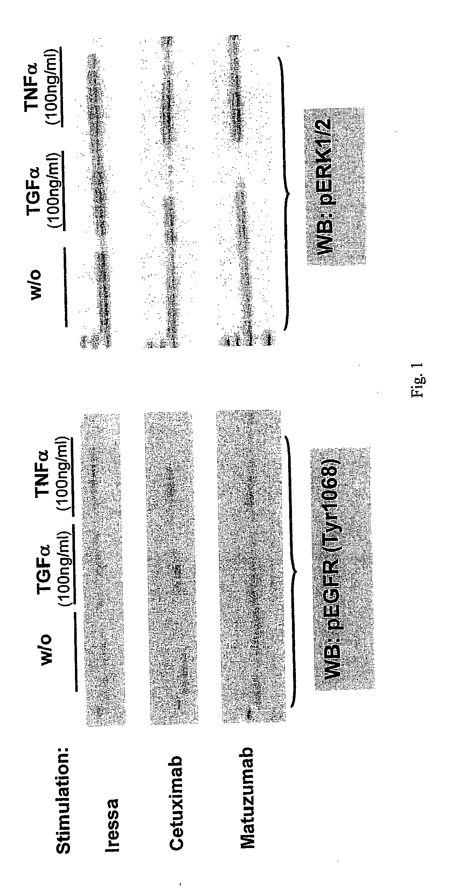

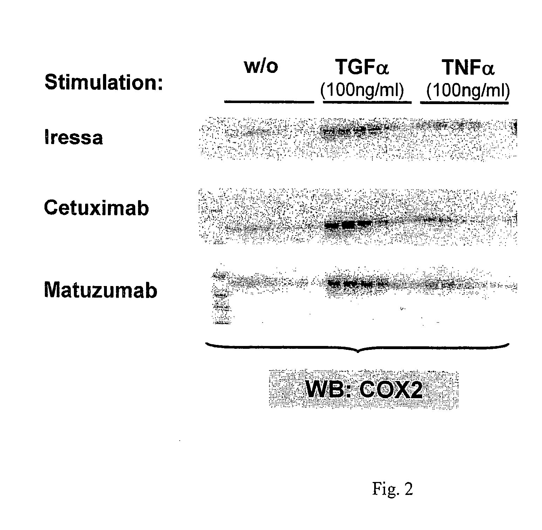

As shown in Figure 1, treatment with anti-EGFR agents interferes with phosphorylation of EGFR and ERK1/2 as measured by Western blot analysis of treated keratinocytes. As shown in Figure 2, treatment with anti-EGFR agents interferes with induction of COX-2 protein. Furthermore, phosphorylation of STAT3 was induced following treatment with anti-EGFR agents.

Experimental work showed that treatment of primary keratinocytes modulates the expression of chemokines *in vitro.* Secreted chemokines were evaluated in conditioned medium of keratinocytes that had been treated with anti-EGFR agents for 24h. Evaluations were carried out with the Luminex bead technology. In these experiments keratinocytes were treated with anti-EGFR inihibitors followed by treatment with or without TGF alpha or TNF alpha.

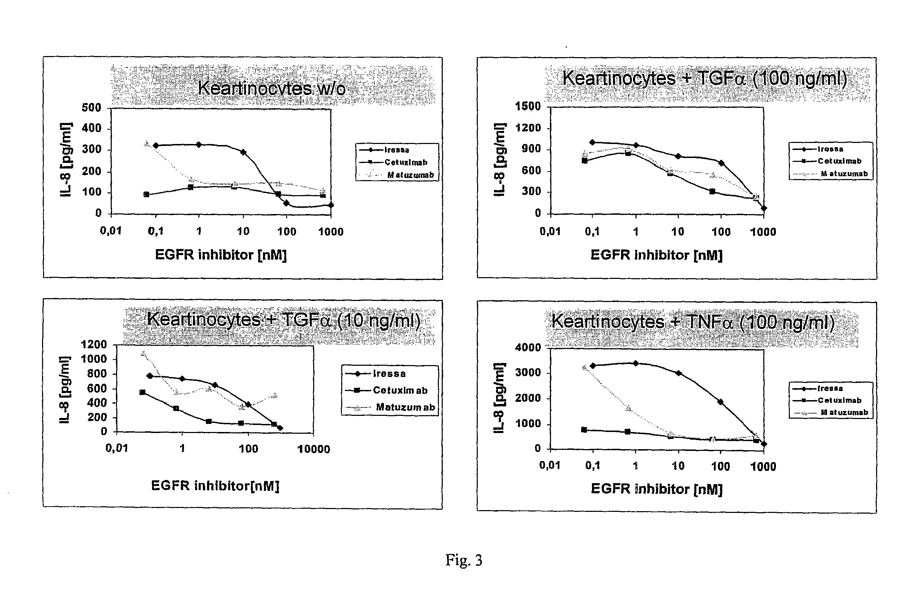

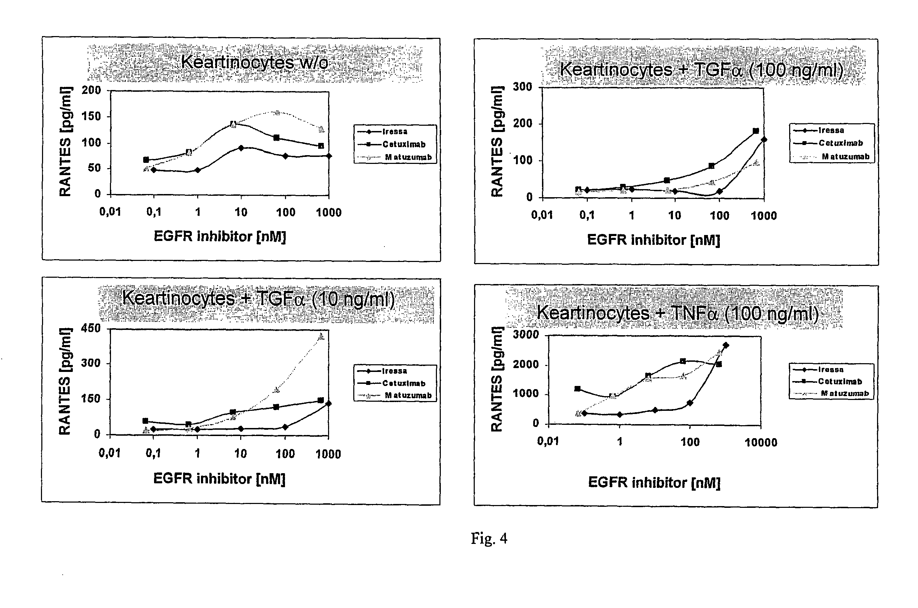

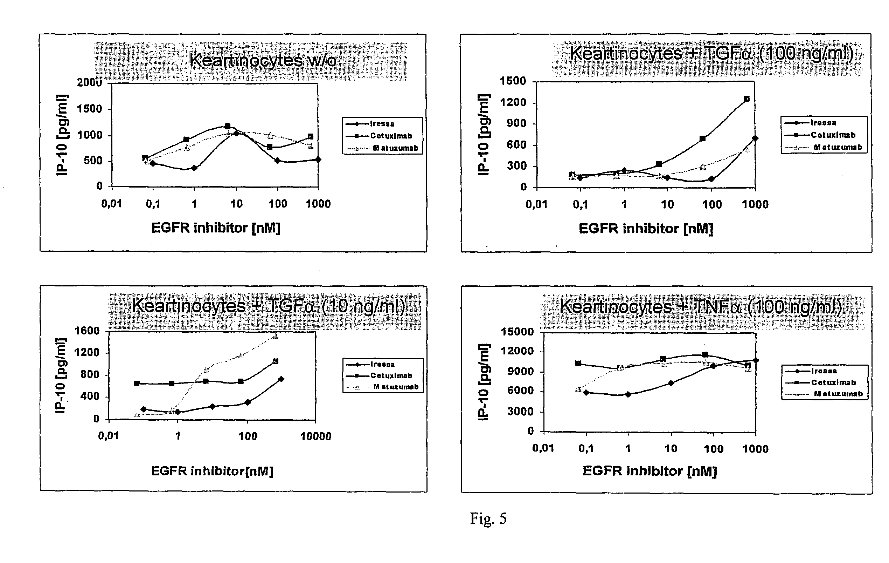

Among several chemokines, IL-8 was consistently down-regulated in response to EGFR blockade (Figure 3), whereas RANTES and IP-10 were up-regulated (Figure 4-5).
IL-8 is a pro-angiogenic factor suggesting that its down-regulation interferes with blood vessel formation in the skin. In contrast, RANTES and IP-10 have been described as chemoattractive factors for leucocytes suggesting that enhanced expression (and possibly also others chemokines) induces infiltration of leucocytes into the skin which cause inflammation and eventually skin rash.
According to the invention, modulated chemokine expression pattern in response to EGFR blockade in keratinocytes and tumor tissue (with active EGFR signaling) results in migration / chemoattraction of leucocytes that can be inhibited by agents/ drugs interfering with these chemokines. Experiments include chemotaxis assays in which conditioned medium of keratinocyte is collected after 24 h of treatment with EGFR inhibitors and stimulation with or without TGF alpha or TNF alpha. The conditioned medium is placed into the lower chamber and freshly islated PBMC or granulocytes are placed into the upper chamber of a Boyden chamber. Chemotaxis of blood cells is then monitored by measuring the amount of PBMC or granulocytes in the lower chamber after speficic times. In some experiments, PBMC or granulocytes are activated in vitro beforehand to enhance the migratory activity of the cells.

According to the invention it could be shown that the chemokines within the conditioned medium cause chemotaxis of PBMC and granulocytes and that this represents part of the biological reaction seen in skin rash.

To show that specific chemokines are responsible for the chemotactic events specific inhibitors to chemokine receptors are added to the conditioned medium into the lower chamber of the Boyden chamber and chemotaxis is evaluated in comparison to conditioned medium only.

Furthermore it has been shown that chemotaxis is induced by chemokine / chemokine receptor interaction and that blockade of this interaction by chemokine receptor anatagonists interferes with chemotaxis of blood cells.

Modulated chemokine expression in response to EGFR blockade results in migration/chemoattraction of leucocytes into the skin of mice. Furthermore, leucocyte infiltration can be analyzed and correlated with chemokine expression.

It has been furthermore shown according to the invention that chemokine levels within the skin of mice are modulated following treatment with anti-EGFR inhibitors, and that this modulation is accompanied by leucocyte infiltration.

Systemic administration of chemokine receptor antagonists have been shown to interfere with leucocyte infiltration into the skin of animals treated with anti-EGFR therapy, and thus reduces/abolishes development of skin rash.

According to the invention individuals can be treated with anti-EGFR agents until first signs of skin toxcitity can bee seen and then the affected diseased skin can be treated topically with anti-chemokine receptor agents to interfere with leucocyte infiltration and reduce skin toxicity.

Topical administration of chemokine receptor antagonists onto the skin reduces leucocyte infiltration and skin toxicity. It is suggested that these agents could be used in clinical practice to treat skin rash of patients undergoing anti-EGFR therapy.

Skin rash has been shown to correlate with the response of patients to anti-EGFR therapy, therefore the expression pattern of chemokines is a more suitable indicator of response than skin toxicity per se and can be used as diagnostic measure to evaluate the patient's responsiveness to anti-EGFR therapy. This can help to pinpoint patients likely to benefit from anti-EGFR therapy within the first week of treatment.

Molecular changes of chemokines levels in the skin of patients can be analyzed within the first week or first ten days following treatment with anti-EGFR antagonists to find out whether chemokines expression is modulated in response to EGFR blockade. This can be done by analyzing skin biopsies prior and on-treatment. Chemokine levels are modulated in response to anti-EGFR therapy and the degree of modulation can be taken as a diagnostic measure to predict if the patient will respond to the treatment. It is suggested that patients that have no or little chemokine modulation are treated at non-optimal doses with anti-EGFR inhibitors, and that doses should be elevated until a modulation is seen. Alternatively, if this is not an option, patients without chemokine modulation could be transferred to a different therapy.

The modulation discovererd by the inventors plays an important role in the development of skin rash in this context and could therefore be used as:
- Diagnostic marker to predict skin rash in tumor patients at early time points
- Surrogate marker of effective target inhibition in tumors (and thereby possibly also clinical outcome), especially to pinpoint patients unlikely to benefit from anti-EGFR therapy shortly after commencement of therapy.
- Development of novel therapies using topical agents that interfere with chemoattraction of chemokines induced by EGFR blockade to manage skin rash.

Modulation of the chemokine milieu of tumors, tumor inflammation and tumor growth inhibition by EGFR inhibitors (cetuximab = c225, matuzumab = EMD72000 = h425) and others.

There are no biomarkers predicting the response of tumor patients to cetuximab therapy. In three indications however - colorectal -, pancreatic - and squamous cell carcinoma of the head - a significant correlation was observed between the degree of acneiform skin rash induced by cetuximab therapy and tumor response. At the standard treatment dose patients presented with no rash and rashes of varying severity (grade I-III) indicating that the degree of the inflammatory process in the skin induced by cetuximab is due to the immune disposition of the individual patients and hence indicating that immunomodulatory activities of cetuximab are an underlying factor contributing to the tumor responses observed. Trafficking and cellular phenotype of immune cells are controlled by chemokines with certain sets of chemokines exhibiting cell type specific activities.

The present invention suggests that the inhibition of EGFR signaling in carcinomas causes changes in the carcinoma chemokine milieu and affects the inflammatory status of the tumor with tumor growth inhibition as consequence.

According to the invention chemokines are regulated in a similar way in tumor cells and primary keratinocytes *in vitro.* As for keratinocytes, experiments were conducted that showed that blocking EGF receptor with monoclonal antibodies, such as cetuximab or EMD72000 or tyrosine kinase inhibitors (gefitinib, Iressa®) interferes with EGFR-dependent signaling cascades in various tumor cell lines such as A431 representing different tumor indications.

Experimental work has shown that treatment of tumor cell lines (such as A431) modulates chemokine expression *in vitro.* Tumor cells were treated with anti-EGFR agents and this was followed by treatment with or without TGF alpha or TNF alpha. Secreted chemokines were evaluated in conditioned medium of tumor cells obtained after 24 h. Evaluation was carried out with the Luminex bead technology.

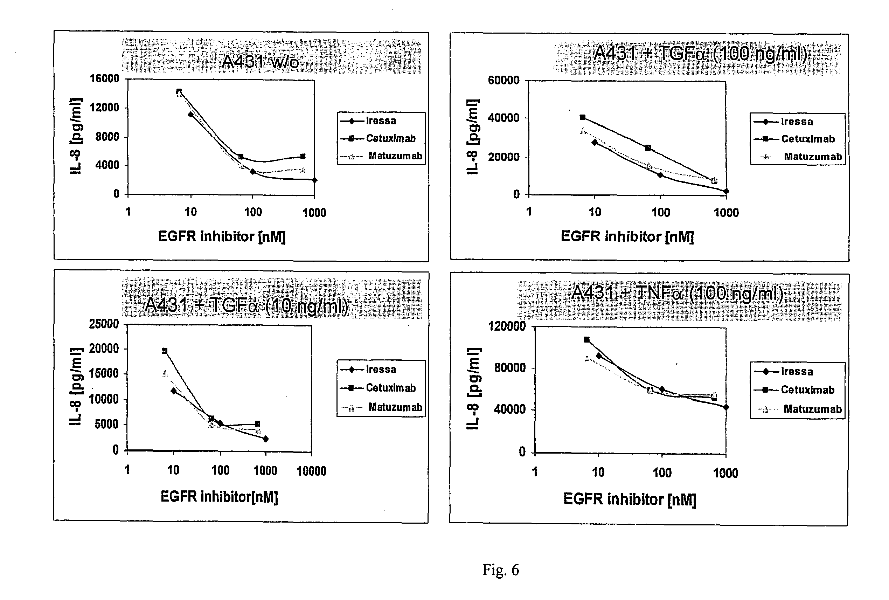

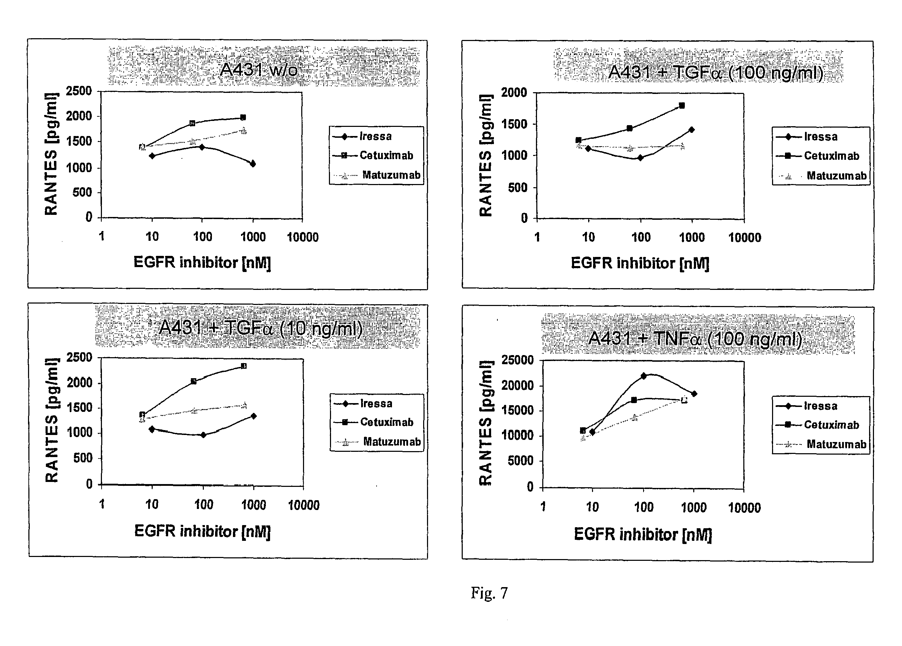

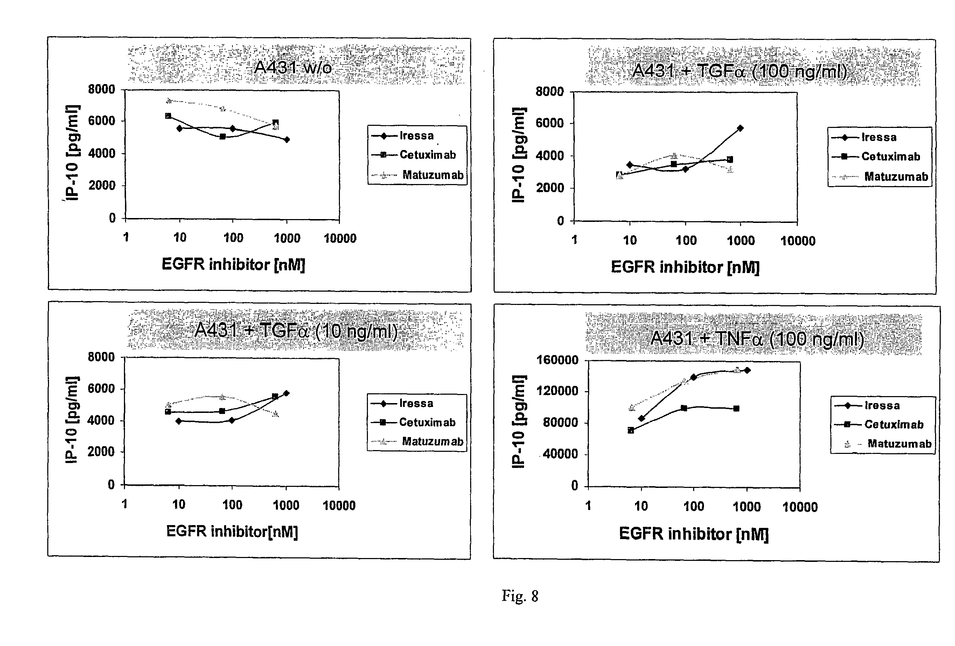

Similar to the data obtained with primary keratinocytes, several chemokines were modulated in response to EGFR blockade in tumor cells. IL-8 was consistently down-regulated (Figure 6), whereas RANTES and IP-10 were up-regulated in tumor cells that were treated with EGFR inhibitors (Figure 7-8).

Together, these data suggest that similar signaling pathways are modulated in keratinocytes and tumor cells by anti-EGFR inhibitors. This corroborates the idea that skin, keratinocytes and more precisely chemokine levels can be used as surrogate to predict effective anti-EGFR therapy in cancer patients.

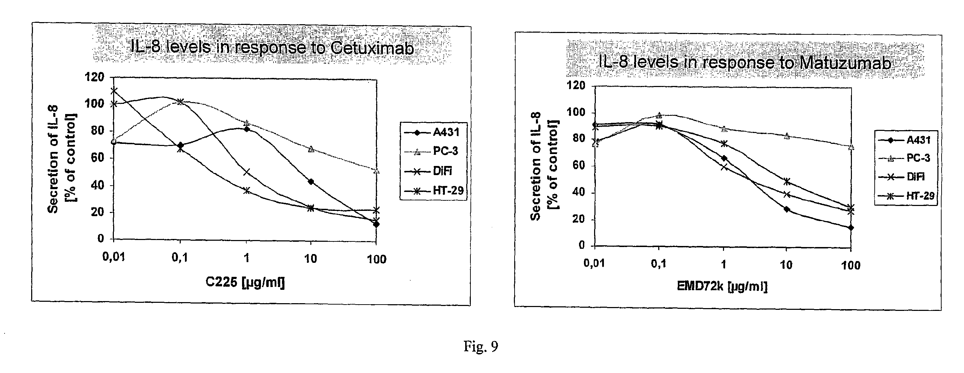

Modulation of IL-8 was evaluated in a panel of different tumor cell lines and was found to be consistently down-regulated in response to EGFR blockade (Figure 9). This suggests that levels of IL-8 are a biomarker of an effective anti-EGFR therapy. It is anticipated to evaluate levels of IL-8 in blood of patients undergoing anti-EGFR therapy, and to use decreased levels as a diagnostic measure to monitor pharmacodynamic effects of anti-EGFR agents.

As mentioned before, modulated chemokine expression pattern in response to EGFR blockade in tumor tissue (with active EGFR signaling) results in migration/chemoattraction of leucocytes.

Experiments include chemotaxis assays in which conditioned medium of tumor cells is collected after 24 h of treatment with EGFR inhibitors and stimulation with or without TGF alpha or TNF alpha. The conditioned medium is placed into the lower chamber and freshly islated PBMC or granulocytes are placed into the upper chamber of a Boyden chamber. Chemotaxis of blood cells is then monitored by measuring the amount of PBMC or granulocytes in the lower chamber after specific times. In some experiments, PBMC or granulocytes are activated in vitro beforehand to enhance the migratory activity of the cells.

These experiments show that the chemokines within the conditioned medium cause chemotaxis of PBMC and granulocytes.

As one result of the invention, specific chemokines, which are responsible for the chemotactic events, specific inhibitors to chemokine receptors are added to the conditioned medium into the lower chamber of the Boyden chamber and chemotaxis is evaluated in comparison to conditioned medium only.

Chemotaxis is induced by chemokine/chemokine receptor interaction and, blockade of this interaction by chemokine receptor anatagonists interferes with chemotaxis of blood cells.

Experiments include in vivo studies in which tumor-bearing mice are treated with anti-EGFR agents. The modulation of chemokine levels within the tumor are analyzed within the first week of treatment with anti-EGFR agents. Chemokines levels are modulated in the same way as observed *in vitro.* Furthermore, leucocyte infiltration are analyzed in tumors, and anti-EGFR agents induce infiltration of leucocytes into the tumor as well as their activity/differentiation status. The latter can be monitored by IHC of cellular activation/differentiation markers. Due to the correlation of skin rash and response to therapy it is suggested that modulation of chemokines takes place in the context of skin as well as cancer, and that this contributes to the mechanism of action of anti-EGFR agents.

According to the invention it is proposed that broad monitoring of changes in chemokine expression profiles in tumors +/- EGFR inhibition and matching the changes observed with the known cellular specificities of chemokines within the immune system provides first clues with respect to which intratumoral chemokines and which leucocytes might contribute to immune control of tumor growth. Based on this information it is possible to identify other therapeutic targets upstream in the pathways controlling chemokine expression beside EGFR that induce immune mediated anti-tumoral effects independent of EGFR inhibition or with the goal to enhance the anti-tumoral effects of anti-EGFR therapeutics.

Thus, it is possible to elucidate alterations in chemokine expression pattern in tumors following EGFR signaling inhibition. The altered chemokine patterns observed under anti EGFR therapy are to a certain degree tumor specific.

### Short Description of the Figures

### Figure 1.

### Inhibition of EGFR-dependent signaling pathways in keratinocytes.

Keratinocytes were treated with EGFR inhibitors (Cetuximab, Matuzumab or Iressa), and stimulated with or without different growth factors (TGFa or TNFa). After 24 h, cells were lysed and analysed by Western blotting. Analyses revealed that treatment with EGFR inhibitors resulted in abrogation of EGFR-driven signaling cascades in a dose-dependent manner. Treatment with EGFR inhibitors prevented phosphorylation of EGFR (Tyr 1068) and ERK1/2 (Thr 202/204).

### Figure 2.

### Inhibition of EGFR-dependent signaling pathways in keratinocytes.

Keratinocytes were treated with EGFR inhibitors (Cetuximab, Matuzumab or Iressa), and stimulated with or without different growth factors (TGFa or TNFa). After 24 h, cells were lysed and analysed by Western blotting. Analyses revealed that treatment with EGFR inhibitors resulted in abrogation of EGFR-driven signaling cascades in a dose-dependent manner. Treatment with EGFR inhibitors induced expression of COX-2 and prevented phosphorylation of STAT3.

### Figure 3.

### Modulation of secreted IL-8 levels in keratinocytes.

Keratinocytes were treated with EGFR inhibitors (Cetuximab, Matuzumab or Iressa), and stimulated with or without different growth factors (TGFa or TNFa). After 24 h, cellular supernatants were collected and levels of IL-8 were quantified using the Luminex technology. Analyses revealed that treatment with EGFR inhibitors down-regulated IL-8 in a dose-dependent manner.

### Figure 4.

### Modulation of secreted RANTES levels in keratinocytes.

Keratinocytes were treated with EGFR inhibitors (Cetuximab, Matuzumab or Iressa), and stimulated with or without different growth factors (TGFa or TNFa). After 24 h, cellular supernatants were collected and levels of RANTES were quantified using the Luminex technology. Analyses revealed that treatment with EGFR inhibitors up-regulated RANTES in a dose-dependent manner.

### Figure 5.

### Modulation of secreted IP-10 levels in keratinocytes.

Keratinocytes were treated with EGFR inhibitors (Cetuximab, Matuzumab or Iressa), and stimulated with or without different growth factors (TGFa or TNFa). After 24 h, cellular supernatants were collected and levels of IP-10 were quantified using the Luminex technology. Analyses revealed that treatment with EGFR inhibitors up-regulated IP-10 in a dose-dependent manner.

### Figure 6.

### Modulation of secreted IL-8 levels in A431.

A431 were treated with EGFR inhibitors (Cetuximab, Matuzumab or Iressa), and stimulated with or without different growth factors (TGFa or TNFa). After 24 h, cellular supernatants were collected and levels of IL-8 were quantified using the Luminex technology. Analyses revealed that treatment with EGFR inhibitors down-regulated IL-8 in a dose-dependent manner.

### Figure 7.

### Modulation of secreted RANTES levels in A431.

A431 were treated with EGFR inhibitors (Cetuximab, Matuzumab or Iressa), and stimulated with or without different growth factors (TGFa or TNFa). After 24 h, cellular supernatants were collected and levels of RANTES were quantified using the Luminex technology. Analyses revealed that treatment with EGFR inhibitors up-regulated RANTES in a dose-dependent manner.

### Figure 8.

### Modulation of secreted IP-10 levels in A431.

A431 were treated with EGFR inhibitors (Cetuximab, Matuzumab or Iressa), and stimulated with or without different growth factors (TGFa or TNFa). After 24 h, cellular supernatants were collected and levels of IP-10 were quantified using the Luminex technology. Analyses revealed that treatment with EGFR inhibitors up-regulated IP-10 in a dose-dependent manner.

### Figure 9.

### Modulation of secreted IL-8 levels in different tumor cell lines.

Different tumor cell lines (DiFi, HT29, A431, MCF-7, PC-3 and U87MG) were treated with EGFR inhibitors (Cetuximab or Matuzumab), and stimulated with TGFa. After 24 h, cellular supernatants were collected and levels of IL-8 were quantified using the Luminex technology. Analyses revealed that treatment with EGFR inhibitors down-regulated IL-8 in all tumor cell lines investigated in a dose-dependent manner.

| **Tumor cells** | **Cmab [% control]** | **Mmab** [% control] |
|---|---|---|
| DiFi | 16 | 28 |
| HT29 | 24 | 30 |
| A431 | 13 | 15 |
| MDA MB 468 | tbd | tbd |
| MCF-7 | 31 | 49 |
| PC-3 | 53 | 76 |
| U87MG | 63 | 58 |

IL-8 levels in response to cetuximab (Cmab) or matuzumab (Mmab).
Cells were treated with 100ng/ml Cmab or Mmab and stimulated with 100ng/ml TGFa.; number of experiments = 1

## Claims

1. An anti-chemokine receptor agent for use for the treatment of skin toxicity or skin rash occurring during anti-EGFR therapy.

2. An anti-chemokine receptor agent of claim 1, wherein the agent is administered until first signs of said skin rash or skin toxicity are observed during anti-EGFR therapy with an anti-EGFR agent.

3. An anti-chemokine receptor agent of claim 1 or 2, wherein said anti-chemokine receptor agent is administered topically onto the skin.

4. An anti-chemokine receptor agent of any of the claims 1 - 3, obtained by determining the chemokine expression pattern presented by the chemokines selected from the group consisting of IL-8, MCP-1, RANTES and IP-10, of a skin biopsy taken from the patient before and 5 - 7 days after onset of the treatment, wherein said anti-chemokine receptor agent is determined from the degree of modulation of the chemokine levels if the patient will respond to the treatment with said ant-EGFR antibody.

5. An in-vitro method of determining the tumor response of a cancer patient to the treatment with an anti-EGFR antibody by determining the chemokine expression pattern presented by the chemokines selected from the group consisting of IL-8, MCP-1, RANTES and IP-10, of a skin biopsy taken from the patient before and 5 - 7 days after onset of the treatment, and predicting from the degree of modulation of the chemokine levels if the patient will respond to the treatment with said ant-EGFR antibody.

6. A method of claim 5, wherein the anti-EGFR antibody is Mab c225 (cetuximab) or Mab h425 (EMD72000, matuzumab).

7. A method of claim 5 or 6, wherein the treatment with the anti-cancer agent causes an increased or reduced expression of chemokines compared to the non-treated patient.
